# EUROPÄISCHE PATENTSCHRIFT

(11) **EP 1 245 556 B1**
(45) Veröffentlichungstag und Bekanntmachung des Hinweises auf die Patenterteilung: **15.09.2004**
(21) Anmeldenummer: 02004922.7
(22) Anmeldetag: 05.03.2002
(51) Int. Cl.: C07C 51/29, C07C 59/64

(54) **Verfahren zur Herstellung von Carbonsäuren durch Oxidation von Aldehyden in Gegenwart von Periodat, Dichromat und Säure in Wasser**
Process for the preparation of carboxylic acids by aldehyde oxidation in presence of periodate, dichromate and acid in water
Procédé de préparation d'acides carboxyliques par oxydation d'aldéhyde et en présence de periodate, dichromate et d'acide dans l'eau

(30) Priorität: 29.03.2001 AT 5062001
(43) Veröffentlichungstag der Anmeldung: 02.10.2002
(73) Patentinhaber: DSM Fine Chemicals Austria Nfg GmbH & Co KG, 4021 Linz (AT)
(72) Erfinder: Alsters, Paul, NL-6224 KZ Maastricht (NL); Schmieder-van de Vondervoort, Elisabeth, NL-6081 AB Haelen (NL)
(74) Vertreter: Lindinger, Ingrid

(56) Entgegenhaltungen:
- WO-A-01/53240
- WO-A-99/52850
- US-A- 4 225 694
- "Methoden der Organischen Chemie : Carbonsäuren und Carbonsäure-Derivate" 1985 , GEORG THIEME VERLAG , STUTTGART XP002201542 5 * Seite 209 - Seite 213 *

## Beschreibung

Die Oxidation stellt eine grundlegende Transformation in der organischen Synthese dar, sodass schon zahlreiche Methoden dafür in der Literatur beschrieben wurden. Trotzdem ist die direkte Umwandlung von Aldehyden zu den entsprechenden Carbonsäuren, insbesondere bei Anwesenheit labiler Substituenten, wie etwa eine gegebenenfalls substituierte Benzylgruppe, noch immer mit Problemen verbunden. Konventionelle Methoden, die Permanganat (Org. Syn., Coll. Vol, 2, 538 (1943)) oder auf Chrom basierende Reagentien J. Org. Chem., 2868 (1967)) verwenden, führen lediglich zu mittelmäßigen Ausbeuten der gewünschten Carbonsäure. Neuere Methoden, die beispielsweise auf der Verwendung von Hypochlorit (Tetrahedron Lett., 23, 3131 (1982)) oder RuCl₃/NaIO₄ (J. Org. Chem., 46, 3936 (1981)) basieren, sind ähnlich erfolglos. Diese Varianten weisen, zusätzlich zu den geringen Ausbeuten, noch den Nachteil auf, dass sehr leicht eine extreme Überoxidation unter C-C-Bindungsbruch auftritt, sodass noch immer nach neuen Oxidationsmethoden gesucht wird.

Aufgabe der Erfindung war es, eine geeignete Methode zur Oxidation von Aldehyden zu den entsprechenden Carbonsäuren zu finden, bei welcher insbesondere auch die Umsetzung bei Anwesenheit labiler Substituenten hoch enolisierbarer Aldehyde in hohem Ausmaß gewährleistet ist.

Unerwarteterweise konnte diese Aufgabe durch die Verwendung von Periodat in Kombination mit Dichromat oder CrO₃ in Anwesenheit einer Säure gelöst werden.

Gegenstand der Erfindung ist demnach ein Verfahren zur Oxidation von Aldehyden zu den korrespondierenden Säuren, das dadurch gekennzeichnet ist, dass ein Aldehyd als Substrat in Gegenwart einer äquimolaren Menge oder eines molaren Überschusses, an Periodat, katalytischer Mengen an Dichromat oder CrO₃ und in Anwesenheit einer Säure in Wasser, einem Wasser/Lösungsmittelgemisch oder in einem Lösungsmittel bei einer Temperatur von -20°C bis +50°C zu der korrespondierenden Säure oxidiert wird.

Bei dem erfindungsgemäßen Verfahren werden Aldehyde zu den korrespondierenden Säuren oxidiert.

Geeignete Aldehyde sind Verbindungen der Formel I

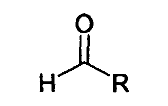

in der R einen linearen, verzweigten oder cyclischen, gegebenenfalls substituierten C₁-C₂₀ Alkylrest, einen Aryl- oder Heteroarylrest oder einen Heterocyclus bedeutet.

Unter Alkylresten sind dabei lineare, verzweigte oder cyclische Alkylgruppen zu verstehen. Diese Reste können dabei unsubstituiert sein oder durch einen oder mehreren unter den Reaktionsbedingungen inerten Substituenten, wie Acyl, Carboxyl, Halogen, C₁-C₈-Alkoxy, C₃-C₈-Cycloalkyl, gegebenenfalls substituierter Arylrest, wie etwa gegebenenfalls durch Phenyl, C₁-C₁₂-Alkyl, C₁-C₈-Alkoxy, Halogen, Aryloxy substituiertes Phenyl oder Naphthyl, Heteroaryl, Heterocyclus u.s.w. substituiert sein.

Unter Aryl sind Phenyl oder Naphthyl zu verstehen, die wiederum unsubstituiert sind oder durch Acyl, Carboxyl, Halogen, C₁-C₈-Alkoxy, C₃-C₈-Cycloalkyl, u.s.w. substituiert sein können.

Heteroarylreste sind dabei 5- oder 6-gliedrige aromatische Ringe, die 1 bis 3 Heteroatome aus der Gruppe O, N oder S aufweisen. Auch diese Reste können unsubstituiert sein oder durch Acyl, Carboxyl, Halogen, C₁-C₈-Alkoxy, C₃-C₈-Cycloalkyl, u.s.w. substituiert sein. Außerdem können die Heteroarylreste als benzokondensierte Ringsysteme vorliegen, die ebenfalls wie oben beschrieben substituiert sein können.

Heterocyclische Reste sind 5- oder 6-gliedrige nichtaromatische Ringe, die 1 bis 3 Heteroatome aus der Gruppe O, N oder S aufweisen. Diese Reste können wiederum unsubstituiert sein oder durch Acyl, Carboxyl, Halogen, C₁-C₈-Alkoxy, C₃-C₈-Cycloalkyl, u.s.w. substituiert sein. Außerdem können auch die heterocyclischen Reste als benzokondensierte Ringsysteme vorliegen, die ebenfalls wie oben beschrieben substituiert sein können.

Bevorzugte Aldehyde sind Aldehyde der Formel I, in der R linearen oder verzweigten, gegebenenfalls substituierten C₁-C₂₀ Alkylrest bedeutet. Bevorzugte Substituenten sind dabei Halogen, C₁-C₈-Alkoxy, Aryloxy oder gegebenenfalls durch C₁-C₁₂-Alkyl, Halogen, C₁-C₈-Alkoxy, Aryloxy substituiertes Phenyl.

Besonders bevorzugt sind Aldehyde der Formel I, in der R einen linearen oder verzweigten C₁-C₂- Alkylrest bedeutet, der durch ein ein- oder zweifach durch C₁-C₄-Alkyl, C₁-C₄-Alkoxy, Phenoxy, Fluor oder Chlor substituiertes Phenyl substituiert ist.

Die erfindungsgemäße Oxidation der Aldehyde erfolgt in Gegenwart einer äquimolaren Menge oder eines molaren Überschusses an Periodat. Bevorzugt werden 1,1 bis 10 Moläquivalente, besonders bevorzugt 1,2 bis 5 Moläquivalente, Periodat verwendet. Periodat wird dabei als Na-, K- oder Bu₄N- salz eingesetzt, wobei Natriumperiodat bevorzugt ist.

Weiters wird für die erfindungsgemäße Oxidation Dichromat oder CrO₃ in katalytischen Mengen zugesetzt. Als Dichromat kommen dabei Na- oder K- dichromat in Frage. Bevorzugt wird Natriumdichromat verwendet. Die Menge an Dichromat bzw. CrO₃ beträgt etwa 0,1 bis 20 mol%, bezogen auf das Substrat. Bevorzugt wird eine Menge von 0,3 bis 3 mol% an Dichromat bzw. CrO₃ zugegeben.

Als dritte Komponente wird eine Säure zugemischt. Als Säure kommen dabei Schwefelsäure, HCl, HNO₃, p-Toluolsulfonsäure (p-TSA), HBF₄, H₅IO₆, CF₃SO₃H oder Perfluortetradecansäure (PFTDA) oder Gemische derselben in Frage. Bevorzugte Säuren sind H₂SO₄, HNO₃ und H₅IO₆, sowie Gemische derselben.

Die Säure wird dabei in einer Menge entsprechend 1-30mol%H⁺, bevorzugt von 5-20mol%H⁺, bezogen auf das Substrat, eingesetzt.

Die erfindungsgemäße Oxidation erfolgt dabei in Wasser, einem Lösungsmittel oder in einem Wasser/Lösungsmittel-Gemisch.
Geeignete Lösungsmittel sind Toluol, Chloroform, Dichlormethan, Ethylacetat, Diethylether, Methyl-t.butylether, Dimethoxyethan, 2-Methoxyethylether, Triethylenglycol-dimethylether, Dioxan, THF, Aceton, i-Propylacetat und Acetonitril.

Bei der Oxidation der Aldehyde werden dabei bevorzugt die drei Oxidationskomponenten Periodat, Dichromat bzw. CrO₃ und Säure in Wasser gelöst. Anschließend wird das zu oxidierende Substrat unter Rühren zugegeben. Das Substrat kann dabei als solches oder gegebenenfalls als Lösung in einem der oben beschriebenen Lösungsmittel oder Wasser/ Lösungsmittelgemisch zugesetzt werden.

Die Reaktionstemperatur beträgt in Abhängigkeit vom gewählten Lösungsmittelsystem -20°C bis +50°C, bevorzugt -10°C bis +30°C und besonders bevorzugt 0°C bis 25 °C.

Wird mit einem Zweiphasensystem gearbeitet, so wird das Reaktionsgemisch während der gesamten Reaktion stark gerührt. Wird nur in wässriger Phase gearbeitet, so kann das starke Rühren gegebenenfalls entfallen.

Die Reaktionsdauer hängt vom eingesetzten Substrat ab und liegt zwischen 1 und 40 Stunden. Bevorzugt liegt die Reaktionszeit zwischen 6 und 30 Stunden, besonders bevorzugt zwischen 12 und 25 Stunden.

Gegebenenfalls kann dem Reaktionsgemisch nach einem Teil der Reaktionszeit eine weitere Portion an Periodat und/oder Säure zugesetzt werden, um die Oxidation zur Carbonsäure zu vervollständigen.

Nach Ende der Oxidation wird die entsprechende Carbonsäure aus dem Reaktionsgemisch isoliert. Dies erfolgt in Abhängigkeit vom Aggregatzustand durch übliche Methoden beispielsweise durch Extraktion, Filtration u.s.w.

Die verbleibende Reaktionslösung kann zur Regenerierung des Periodates aufgearbeitet werden. Dies kann durch literaturbekannte Methoden, beispielsweise durch chemische oder elektrochemische Oxidation erfolgen. Bevorzugt erfolgt die Regenerierung des Periodat durch Ozon, wie etwa in WO 98/27118 beschrieben. Das regenerierte Periodat kann sodann für weitere Oxidationen wieder eingesetzt werden.

Durch das erfindungsgemäße Verfahren werden Aldehyde zu den korrespondierenden Carbonsäuren je nach Reaktionsdauer bis zu einem Prozentsatz von 70% und darüber umgesetzt. Unumgesetzte Aldehyde lassen sich dabei leicht vom Endprodukt bei dessen Isolierung abtrennen.
Ein weiterer Vorteil des Verfahrens ist die einfache Reaktionsdurchführung, wobei insbesondere von Vorteil ist, dass auch Aldehyde mit labilen Substituenten, wie etwa einer gegebenenfalls substituierten Benzylgruppe, in im Vergleich zum Stand der Technik hohen Ausbeuten und hoher Reinheit erhalten werden.

### Beispiel 1: p-Methoxyphenylessigsäure

1.44 Äquivalente (7.7g) Natriumperiodat NalO₄, 0,6mol% (0.045g) Natriumdichromat Na₂Cr₂O₇·2H₂O und 3g 1 N Schwefelsäure H₂SO₄ (12mol%) wurden in 50ml Wasser gelöst. Zu dieser Lösung wurden 25 mmol (3,75g) 4-Methoxyphenylacetaldehyd und 25ml Toluol zugesetzt, worauf das Reaktionsgemisch 17h bei 20°C gerührt wurde. Anschließend wurden die Phasen getrennt und die wässrige Phase mit 2x10ml Toluol extrahiert. Die vereinigten Toluolphasen wurden mit 30ml 1 N NaOH versetzt. Die basische Wasserphase wurde mit 3g konz. H₂SO₄ sauer gestellt und mit 2x25ml Toluol extrahiert. Die vereinigten organischen Phasen wurden 3x mit H₂O gewaschen, über Na₂SO₄ getrocknet und eingedampft. Es wurden 17mmol (2,82g) p-Methoxyphenylessigsäure (Ausbeute 68% d.Th.) mit einer Reinheit von 98% erhalten.

## Patentansprüche

1. Verfahren zur Oxidation von Aldehyden zu den korrespondierenden Säuren, **dadurch gekennzeichnet, dass** ein Aldehyd als Substrat in Gegenwart einer äquimolaren Menge oder eines molaren Überschusses, an Periodat, katalytischer Mengen an Dichromat oder CrO₃ und in Anwesenheit einer Säure in Wasser, einem Wasser/Lösungsmittelgemisch oder in einem Lösungsmittel bei einer Temperatur von -20°C bis +50°C zu der korrespondierenden Säure oxidiert wird.

2. Verfahren nach Anspruch 1, **dadurch gekennzeichnet, dass** als Aldehyde Verbindungen der Formel I 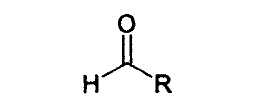 in der R einen linearen, verzweigten oder cyclischen, gegebenenfalls substituierten C₁-C₂₀-Alkylrest, einen Aryl- oder Heteroarylrest oder einen Heterocyclus bedeutet, eingesetzt werden.

3. Verfahren nach Anspruch 2, **dadurch gekennzeichnet, dass** als Aldehyde Verbindungen der Formel I, in der R einen linearen oder verzweigten, gegebenenfalls durch Halogen, C₁-C₈-Alkoxy, Aryloxy oder durch gegebenenfalls mit C₁-C₁₂-Alkyl, Halogen, C₁-C₈-Alkoxy, Aryloxy substituiertes Phenyl substituierten C₁-C₂₀ Alkylrest bedeutet, eingesetzt werden.

4. Verfahren nach Anspruch 2, **dadurch gekennzeichnet, dass** Aldehyde der Formel I, in der R einen linearen oder verzweigten C₁-C₂-Alkylrest bedeutet, der durch ein ein- oder zweifach durch C₁-C₄-Alkyl, C₁-C₄-Alkoxy, Phenoxy, Fluor oder Chlor substituiertes Phenyl substituiert ist, eingesetzt werden.

5. Verfahren nach Anspruch 1, **dadurch gekennzeichnet, dass** 1,1 bis 10 Moläquivalente an Periodat zugesetzt werden.

6. Verfahren nach Anspruch 1, **dadurch gekennzeichnet, dass** Periodat in Form des Na-, K- oder Bu₄N- Salzes eingesetzt wird.

7. Verfahren nach Anspruch 1, **dadurch gekennzeichnet, dass** Dichromat oder CrO₃ in einer Menge von 0,1 bis 20 mol%, bezogen auf den Aldehyd eingesetzt wird.

8. Verfahren nach Anspruch 1, **dadurch gekennzeichnet, dass** als Säure Schwefelsäure, HCl, HNO₃, p-Toluolsulfonsäure, HBF₄, H₅IO₆, CF₃SO₃H oder Perfluortetradecansäure oder Gemische derselben eingesetzt werden.

9. Verfahren nach Anspruch 8, **dadurch gekennzeichnet, dass** eine Menge an Säure entsprechend 1-30mol%H⁺, bezogen auf das Substrat, eingesetzt wird.

10. Verfahren nach Anspruch 1, **dadurch gekennzeichnet, dass** die Reaktion in Wasser, einem Lösungsmittel aus der Gruppe Toluol, Chloroform, Dichlormethan, Ethylacetat, Diethylether, Methyl-t.butylether, Dimethoxyethan, 2-Methoxyethylether, Triethylenglycol-dimethylether, Dioxan, THF, Aceton, i-Propylacetat und Acetonitril oder in einem Wasser/Lösungsmittel-Gemisch durchgeführt wird.

## Claims

1. A process for the oxidation of aldehydes to the corresponding acids, which comprises oxidizing an aldehyde as substrate to the corresponding acid in the presence of an equimolar amount, or a molar excess, of periodate, catalytic amounts of dichromate or CrO₃, and in the presence of an acid in water, a water/solvent mixture or in a solvent at a temperature of from -20°C to +50°C.

2. The process as claimed in claim 1, wherein the aldehydes are compounds of the formula I 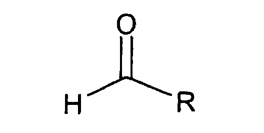 where R is an unbranched, branched or cyclic, unsubstituted or substituted C₁-C₂₀-alkyl radical, an aryl radical or heteroaryl radical or a heterocycle.

3. The process as claimed in claim 2, wherein the aldehydes are compounds of the formula I where R is an unbranched or branched C₁-C₂₀-alkyl radical which is unsubstituted or substituted by halogen, C₁-C₈-alkoxy, aryloxy or phenyl which is unsubstituted or substituted with C₁-C₁₂-alkyl, halogen, C₁-C₈-alkoxy, aryloxy.

4. The process as claimed in claim 2, wherein the aldehydes are compounds of the formula I where R is an unbranched or branched C₁-C₂-alkyl radical which is substituted by a phenyl which is monosubstituted or disubstituted by C₁-C₄-alkyl, C₁-C₄-alkoxy, phenoxy, fluorine or chlorine.

5. The process as claimed in claim 1, wherein from 1.1 to 10 molar equivalents of periodate are added.

6. The process as claimed in claim 1, wherein periodate is used in the form of the Na, K or Bu₄N salt.

7. The process as claimed in claim 1, wherein dichromate or CrO₃ is added in amounts of from 0.1 to 20 mol%, based on the aldehyde.

8. The process as claimed in claim 1, wherein the acid is sulfuric acid, HCl, HNO₃, p-toluenesulfonic acid, HBF₄, H₅IO₆, CF₃SO₃H or perfluorotetradecanoic acid or mixtures thereof.

9. The process as claimed in claim 8, wherein an amount of acid corresponding to 1-30 mol% H⁺, based on the substrate, is used.

10. The process as claimed in claim 1, wherein the reaction is carried out in water, a solvent selected from the group consisting of toluene, chloroform, dichloromethane, ethyl acetate, diethyl ether, methyl t-butyl ether, dimethoxyethane, 2-methoxyethyl ether, triethylene glycol dimethyl ether, dioxane, THF, acetone, isopropyl acetate and acetonitrile, or in a water-solvent mixture.

## Revendications

1. Procédé pour l'oxydation d'aldéhydes en acides correspondants, **caractérisé en ce qu'**un aldéhyde est oxydé, en tant que substrat, en un acide correspondant, en présence d'une quantité équimolaire ou d'un excédent molaire de périodates, d'une quantité catalytique de bichromate ou de CrO₃ et en présence d'un acide, dans de l'eau, dans un mélange à l'eau ou de solvants, ou dans un solvant, à une température allant de -20°C à +50°C.

2. Procédé selon la revendication 1, **caractérisé en ce que** des composés de formule I sont utilisés comme aldéhydes 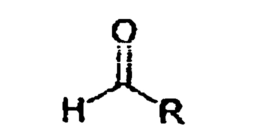 dans laquelle R représente un radical alkyle en C₁-C₂₀ linéaire, ramifié ou cyclique, le cas échéant substitué, un radical aryle ou hétéro aryle ou un hétérocycle.

3. Procédé selon la revendication 2, **caractérisé en ce que** des composés de formule I sont utilisés comme aldéhydes, dans laquelle R représente un radical alkyle en C₁-C₂₀ linéaire ou ramifié, le cas échéant substitué par un halogène, un alcoxy en C₁-C₈, un aryloxy ou le cas échéant par un phényle substitué par un alkyle en C₁-C₁₂, un halogène, un alcoxy en C₁-C₈, ou un aryloxy.

4. Procédé selon la revendication 2, **caractérisé en ce que** des aldéhydes de formule I sont utilisés, dans laquelle R représente un radical alkyle en C₁-C₂ linéaire ou ramifié, qui est substitué par un phényle simplement ou doublement substitué par un alkyle en C₁-C₄, un alcoxy en C₁-C₄, un phénoxy, du fluor ou du chlore.

5. Procédé selon la revendication 1, **caractérisé en ce que** 1,1 à 10 équivalents molaires de périodates sont ajoutés.

6. Procédé selon la revendication 1, **caractérisé en ce que** le périodate est utilisé sous la forme d'un sel de Na, K ou Bu₄N.

7. Procédé selon la revendication 1, **caractérisé en ce que** du bichromate ou du CrO₃ est utilisé dans une quantité allant de 0,1 à 20 mol% en se rapportant à l'aldéhyde.

8. Procédé selon la revendication 1, **caractérisé en ce que** de l'acide sulfurique, de l'HCl, de l'HNO₃, de l'acide p-toluène sulfonique, de l'HBF₄, de l'H₅IO₆, du CF₃SO₃H ou un acide perfluorotétradécanoique ou des mélanges de ceux-ci sont utilisés comme acide.

9. Procédé selon la revendication 8, **caractérisé en ce qu'**une quantité d'acide correspondant à 1-30 mol%H⁺ est utilisée en se rapportant au substrat.

10. Procédé selon la revendication 1, **caractérisé en ce que** la réaction est effectuée dans de l'eau, dans un solvant du groupe toluène, chloroforme, dichloro méthane, acétate d'éthyle, diéthyléther, éther de méthyle-t-butyle, diméthoxyéthane, éther de 2-méthoxyéthyle, triéthylèneglycol-diméthyléther, dioxanne, THF, acétone, acétate d'i-propyle et nitrile d'acétone ou dans un mélange à l'eau ou de solvants.
